# EUROPEAN PATENT APPLICATION

(11) **EP 1 413 274 A1**
(43) Date of publication of application: **28.04.2004**
(21) Application number: 02771754.5
(22) Date of filing: 21.05.2002
(51) Int. Cl.: A61F 13/15

(54) **INTERLABIAL PAD**

(30) Priority: 22.05.2001 JP 2001152403; 03.09.2001 JP 2001265966
(71) Applicant: UNI-CHARM CORPORATION, Kawanoe-shi Ehime 799-0111 (JP)
(72) Inventor: MIZUTANI, S., Technical Center, Uni-Charm Corp., Mitoyo-gun, Kagawa 769-1602 (JP); YAMAKI, K., Technical Center, Uni-Charm Corp., Mitoyo-gun, Kagawa 769-1602 (JP); NODA, Y., Technical Center, Uni-Charm Corp., Mitoyo-gun, Kagawa 769-1602 (JP); TOKUMOTO, M., Technical Center, Uni-Charm Corp., Mitoyo-gun, Kagawa 769-1602 (JP); SAKAI, A., Technical Center, Uni-Charm Corp., Mitoyo-gun, Kagawa 769-1602 (JP)
(74) Representative: Purdy, Hugh Barry
(86) International application number: PCT/JP2002/004894
(87) International publication number: WO 2002/094158

(57) **Abstract**

The present invention relates to an interlabial pad which is attached between female labia, especially to the pad which can be used together with the sanitary napkin, further to a wrapping body where said interlabial pad is contained individually in a wrapping container, and the present invention provides an interlabial pad in capable of rapidly directing the menstrual blood which flows down along the inner wall of labia to an absorbent body and in capable of improving the shape fitness (fitness ability) within labia.

The interlabial pad (14) comprises the absorbing sheet and the support sheet (12) backing the absorbing sheet. The absorbing sheet is formed as an aggregate juxtaposing a plurality of strip-type absorbing areas, the absorbing area includes an absorbent body (13a, 13b) which is individually contained in a cover sheet (11a, 11b), and the strip-type absorbing area is formed into an area (14a) contacting with the neighborhood of the ostium vaginae of the user and an area (14b) contacting with an inner wall of labia of the user.

## Description

### Background of the Invention

### Technical Field

The present invention relates to an interlabial pad, which is used by fitting between female labia, and particularly relates to the interlabial pad, which can be used together with a sanitary napkin, and relates to a wrapping body comprising the interlabial pad is contained in a wrapping container individually.

### Background Art

Conventionally, a sanitary napkin and a tampon are used generally as sanitary products for female. However, as for the sanitary napkin used by fixing it to a garment, it tends to cause menstrual blood leakage from a gap made from a poor contact with a vicinity of the ostium vaginae. It tends to cause the foreign feeling and the discomfort as for the tampon, on the basis of the nature of its products when wearing it, and it is difficult to insert it into the vagina.

Under such situation, an interlabial pad of a sanitary product has attracted people as a sanitary product positioned between the sanitary napkin and the tampon in recent years.

The interlabial pad is used by inserting a partial portion of it between the labia and being brought into contact with the labia. It has better wear feeling and is more comfortable because of its small size as compared with the sanitary napkin. It is sanitary and clean because menstrual blood may smear a small range of the body. Further, it is characterized in that a closer contact with the body than the sanitary napkin does may prevent menstrual blood leakage and that it gives a lower psychological resistance to wear it than the tampon which is inserted into the vagina.

However, the conventional interlabial pad has had a problem that the menstrual blood flows over the pad surface and leaks out of the pad surface before it is absorbed by the absorbent body. Since the menstrual blood flows along the inner wall of labia at the slow speed with a high degree of wettability to the inner wall of the labia, a large amount of retained menstrual blood between the labia flows out all at once of the labia, Instead, it flows out of the labia gradually.

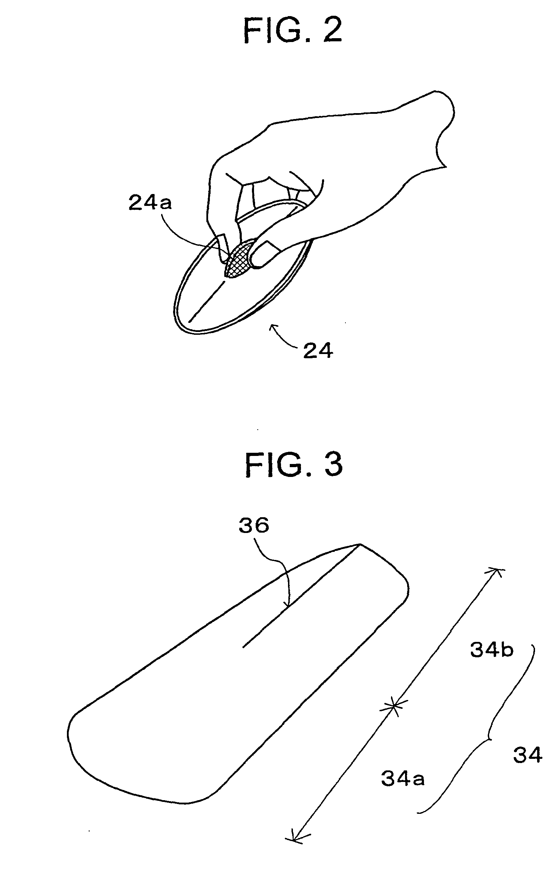

For example, as illustrated in Fig. 2, the interlabial pad 24 has a knob portion 24a at the side of the garment, thereby the person can wear it easily by holding the knob 24a with fingers (International Publication No. WO99/56689). However, the pad disclosed in the publication is lack of adhesion to the inner wall of the labia since it is difficult to apply pushing force to a portion other than near the knob to push the pad between the labia. Therefore, before the absorbent body absorbs the menstrual blood, the menstrual blood flows over the pad surface and leaks out of the pad.

Further as illustrated in Fig. 3, an interlabial pad 34 is designed that a convex portion can be formed by folding a rear side area 34b along a substantial center line 36 in the longitudinal direction of the pad. As compared with the interlabial pad 24 illustrated in Fig. 2, this interlabial pad 34 can be used by putting the convex portion between labia and have improved contact state with the labia inner wall. However, although the pad has such a structure as described hereinbefore, the menstrual blood flows easily out of the pad surface and menstrual blood leakage may occur. Therefore, due to the smaller size of the interlabial pad than a sanitary napkin, the serious damage by the menstrual blood leakage may be caused.

### Disclosure of the Invention

With respect to problems described hereinbefore, it is an object of the present invention to provide an interlabial pad capable of quickly directing the menstrual blood, which flows along the inner wall of the labia, to its absorbent body.

To solve the aforesaid problems, the interlabial pad preferably comprises an absorbing sheet which is formed as an aggregate juxtaposing a plurality of strip-type absorbing areas including an absorbent body, which is independently contained in a cover sheet, wherein the strip-type absorbing area comprises an area 14a which contacts around ostium vaginae of the user and an area 14b which contacts an inner wall of the user's labia, so that the menstrual blood which flows along the inner wall of the labia is rapidly absorbed by the absorbent body and that the shape fitness (fitting ability) in labia can be improved.

Concretely the present invention provides the interlabial pad as follows;

(1) An interlabial pad which is used by fitting between user's labia comprising an absorbing sheet portion facing a body side when the pad is worn and including an absorbent body that absorbs body fluid, and a support sheet backing the absorbing sheet portion;
wherein the absorbing sheet portion is formed as an aggregate juxtaposing a plurality of strip-type absorbing areas in a belt shape which include independent absorbent bodies; and wherein the strip-type absorbing area disposed on a central portion of the absorbing sheet portion forms a body fluid discharge port contact area that contacts a vicinity of a body fluid discharge port of the user; and wherein the strip-type absorbing areas disposed on both outwardly-folded sides of the absorbing sheet portion form a labia inner wall contact area that contacts an inner wall of the user's labia.

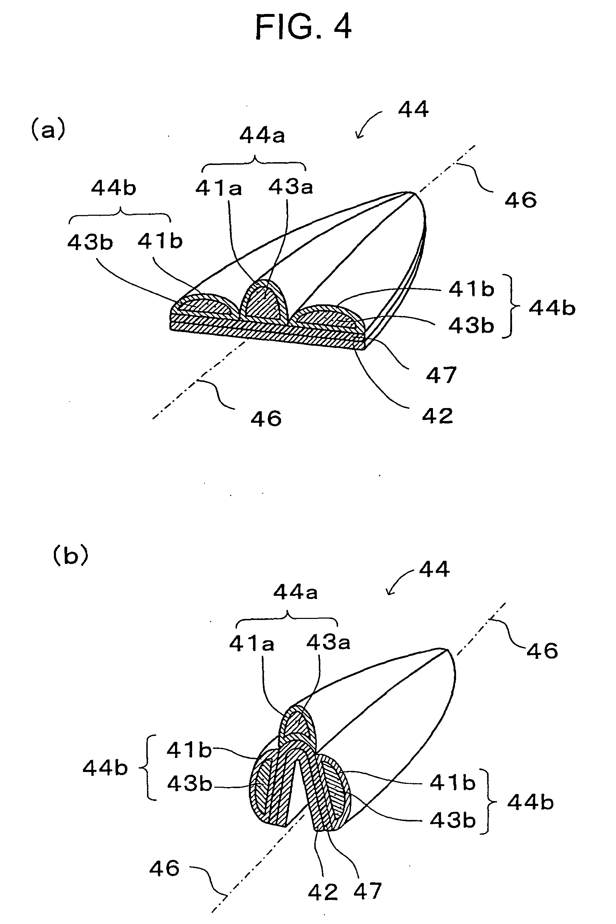

In the interlabial pad of the present invention, for example as shown in Fig.4 (a), the pad comprises the absorbing sheet portion and the support sheet 42 backing the absorbing sheet portion. The absorbing sheet portion contains the absorbent body to absorb the body fluid such as menstrual blood and is used to face the body side. Further, the absorbing sheet portion is formed as an aggregate juxtaposing a plurality of (three as illustrated) strip-type absorbing areas, which include independent absorbent bodies 43a, 43b, respectively.

In the plurality of strip-type absorbing areas, an ostium vaginae contacting area 44a is formed on the area disposed on the center portion of the absorbing sheet portin(along the substantial center line 46 in the longitudinal direction as illustrated) so as to contact with the ostium vaginae of the user. Further on the strip-shape absorbing areas, which are disposed on both outwardly-foldedsides of the absorbing sheet portion, each labia inner wall contacting area 44b is formed to contact each labia inner wall of the user.

As illustrated in Fig. 4(b), when the interlabial pad 44 is folded along the substantial center line 46 and is inserted between labia, the adhere condition is so good that the fluid discharge port, for example, an ostium vaginae contacting area 44a, contacts with a deep portion of labia including an ostium vaginae vicinity and that each labia inner wall contact area 44b contacts each inner wall of labia closely. Therefore, most menstrual blood discharged from the ostium vaginae is absorbed firstly by the ostium vaginae contact area 44a which contacts closely with the deep portion of labia including the ostium vaginae vicinity. Even if a large amount of the menstrual blood flows out at once, it is absorbed secondarily by the area 44b which contacts the inner wall of labia. As described hereinbefore, a large amount of menstrual blood, which flow down at once along the labia inner walls, can be directed rapidly by a combination of the ostium vaginae contact area 44a and the labia inner wall contact area 44b. Further the area 44a and the area 44b, respectively, include absorbent bodies 43a, 43b, which are independently contained in the cover sheet 41a, 41b. Thereby, the strip-type absorbent bodies are easily folded so as to have a good shape shiftness. Then, the shape fitness (fittability) in the labia is improved.

(2) The interlabial pad according to (1), wherein a side end of the labia inner wall contact area comprises an arc-shaped portion and wherein both edge ends of the arch-shaped portion converge toward edge ends in a longitudinal direction of the body fluid discharge port contact area.

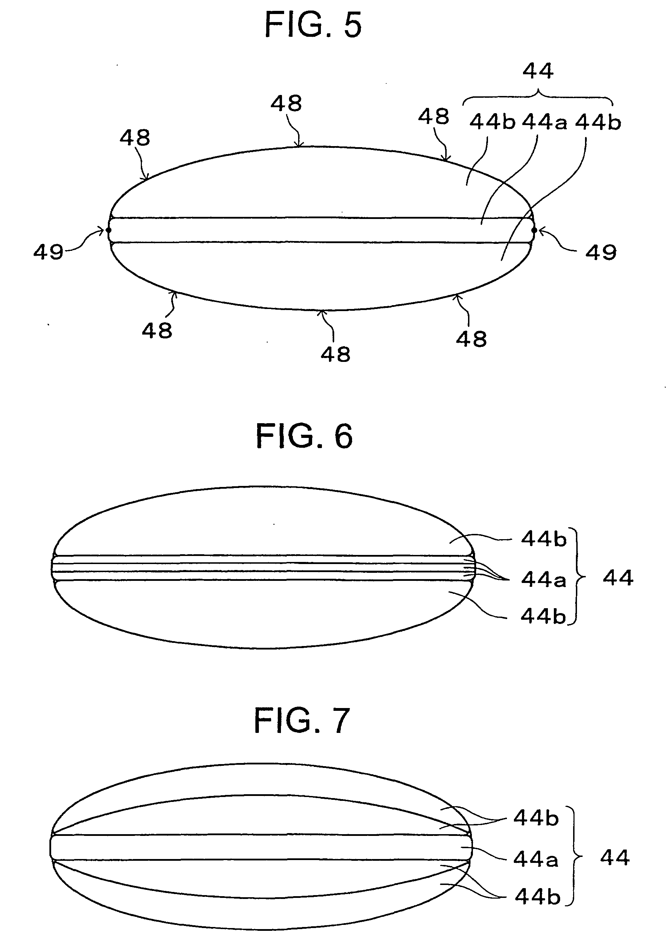

It is not limited to the specific whole shape of the interlabial pad of the present invention and any kind of shape may be employed if it fits to labia. For example, an elliptic type, an ovoid, a gourd-shape or a drop-shape and the like may be used. However in consideration of the shape fitness with labia, as shown in Fig. 5, preferably a side end 48 of the labia inner wall contact area 44b comprises an arc-shaped portion and both edges of the arch-shaped portion converge toward edge ends 49 in the longitudinal direction of the ostium vaginae contact area 44a.

(3) The interlabial pad according to (1) or (2), wherein the body fluid discharge port contact area and/or the labia inner wall contact area is divided into a plurality of portions.

In the interlabial pad of the present invention, the ostium vaginae contact area or the labia inner wall contact area may be divided into a plurality of portions. For example, as illustrated in Fig. 6, a plurality of ostium vaginae contact areas 44a are divided into a plurality of portions (three as illustrated), or as illustrated in Fig. 7, a plurality of labia inner-wall contact areas 44b can be divided into a plurality of portions (two at the both sides as illustrated). Of course, both ostium vaginae contact area and labia inner wall contact area may be divided into a plurality of portions. Compared with the configuration, where each area is not divided, these configurations are preferable to increase the shape fitness (fittability) in labia. Further absorbent bodies contained in each area are accordingly divided. Thereby, a respective absorbent body can absorb the menstrual blood adequately in each place to achieve effective absorption.

(4) The interlabial pad according to any one from (1) to (3) comprising a groove portion between the body fluid discharge port contact area and the labia inner wall contact area, or between the body fluid discharge port contact area divided into a plurality of portions and the labia inner wall contact area divided into a plurality of portions.

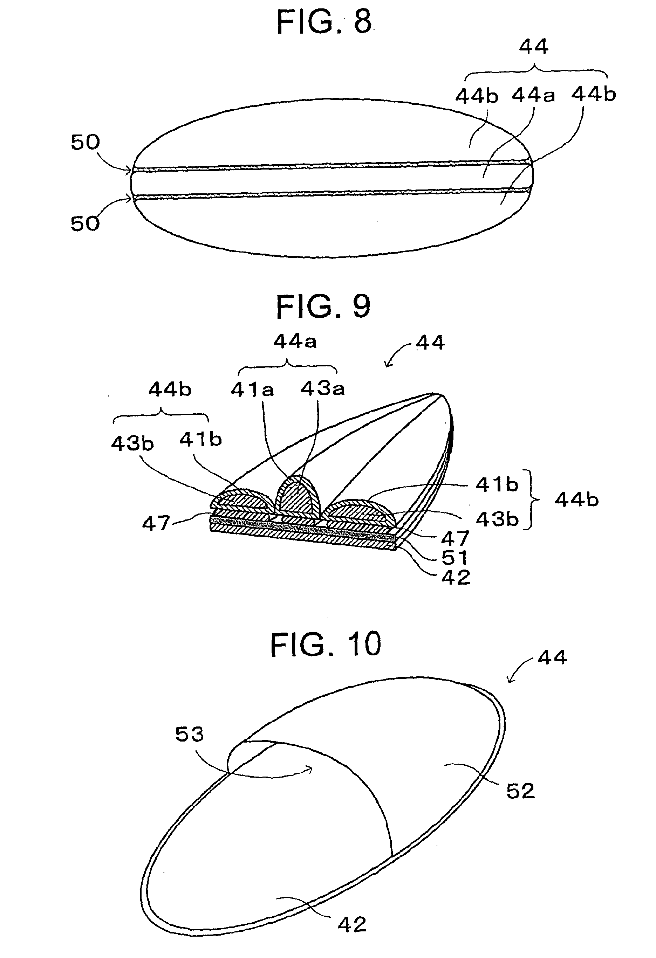

In the interlabial pad of the present invention, it is not limited to the configuration that ostium vaginae contact area is next to the labia inner wall contact area. But a groove portion may be provided between the ostium vaginae contact area and the labia inner wall contact area. For example, as shown in Fig. 8, the groove portion 50 can be provided between them if the distance from 1 to 5mm between the area 44a and the area 44b is made.

In a normal interlabial pad, after the menstrual blood passes through the cover sheet once, a part of the menstrual blood solidifies and remains in the cover sheet. Therefore, even if the absorbent body still has an ability to absorb the menstrual blood, it has a nature not to absorb the menstrual blood any more. In the case of providing the groove portion 50 as described hereinbefore, the menstrual blood flows rapidly along the groove portion 50. Therefore, the absorbent body of the ostium vaginae contact area 44a and the labia inner wall contact area 44b can be used effectively and performs its best absorption capacity. Further as described in aforesaid (3), in the case of dividing the ostium vaginae contact area and the labia inner wall contact area into plurality portions, the same effect can be achieved by providing the groove portion between the portions of the ostium vaginae contact area or the portions of the labia inner wall contact area.

(5) The interlabial pad according to any one from (1) to (4) comprising an inner sheet having water permeability or water-absorbability and being inserted between a garment side face of the absorbing sheet and a body side face of the support sheet.

In the interlabial pad according to the present invention, for example, as illustrated in Fig. 9, between a garment side face of the absorbing sheet (i.e., cover sheet 41a, 41b and absorbent body 43a, 43b) and a body side face of the support sheet 42, is preferable to insert an inner sheet 51 having water permeability or water absorbability. By forming such structure, even if a larger amount of menstrual blood than what the ostium vaginae contact area 44a can firstly absorb is discharged from the ostium vaginae, the menstrual blood may pass through the inner sheet 51 into labia inner wall contact area 44b. Therefore, the pad can absorb a large volume of menstrual blood with a rapid flow rate.

In this case, it is more preferable that the fiber constituting the inner sheet 51 is oriented in the lateral direction of the interlabial pad 44, since the absorbed menstrual blood easily moves in the oriented direction of fiber layer, i.e., a left-and-right direction of the body so that the menstrual blood may move from the ostium vaginae contact area 44a to the labia inner wall contact area 44b rapidly.

(6) The interlabial pad according to any one from (1) to (5) comprising a mini sheet piece provided and bonded on the garment side face of the support sheet that has one or more bonding portions at each side portion in a longitudinal direction of the support sheet; and has one or more non-bonding portions in a lateral direction of the support sheet; and wherein between the mini sheet piece and the support sheet, at least one non-bonding portions form a finger insertion opening through which a finger can be inserted so that an opening size of the finger insertion opening is directly maintained as large as a finger breadth in a surface direction of the support sheet.

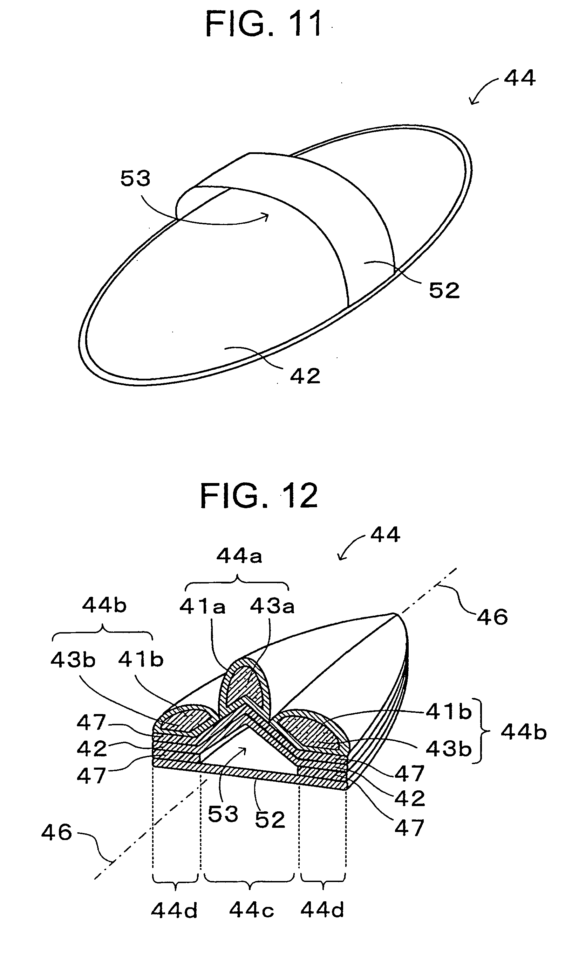

In the interlabial pad according to the present invention, for example as illustrated in Fig. 10 or 11, a mini sheet piece 52 is disposed to form a finger insertion opening 53. In Fig. 10 and 11, in a lateral direction of the support sheet 42, at least one of both sleeve portions of the mini sheet piece 52 is not bonded with the support sheet 42 surface. Thereby, an opening is formed between one sleeve side of the mini sheet piece 52 which is in a non-bonding condition and the support sheet 42 to form a finger insertion opening 53 which is capable of receiving an inserted finger.

In a longitudinal direction of the support sheet 42, the mini sheet piece 52 is connected with only both lateral sides of the support sheet 42 and is not bonded (adhered) with the inside thereof. Therefore, the mini sheet piece 52 is provided from one lateral side of the support sheet 42 to the other lateral side thereof and bonded at each end so that an overpass portion extends from one lateral side to the other, to form a penetrating or non-penetrating space (space for finger insertion). Such space can be retained by inserting a finger.

The word "finger breadth" of this specification does not mean the thickness of the finger. But the width of the finger in the spread direction of the nail in concrete. The opening size as large as the finger breadth means that it has a sufficient size for finger insertion.

The opening size as large as the finger breadth is directly kept in a direction of the support sheet surface. This means that when the finger is inserted into the pad normally to wear the pad, the finger is inserted with its finger cushion directed to the garment face side of the support sheet, the pad itself forms its shape to have an adequate portion for finger insertion preliminarily. Therefore, the following case is an exception from the above described examples. The opening for the finger breadth is made in a surface direction by turning the finger after the user inserts the finger so that the opening size is rendered on the support sheet surface side secondarily.

As described hereinbefore, in the pad provided with the mini sheet piece, the finger is inserted into the opening through. Thereby, the pad can be kept and fixed at the finger for the time being. In this case, the finger insertion opening is formed to be the opening size as large as the finger breadth, and the flat-shaped fingertip is inserted without being aligned to the different direction with respect to the support sheet so as to be inserted to contact the support sheet surface naturally. That is, the finger insertion opening has a wide shape in a direction of the support sheet surface in accordance with the shape of the fingertip of the wearing person, therefore the user is intended to act that the direction where the finger is inserted is determined to detect a fitting point of the pad by the finger tip. Thereby, even between labia where it is difficult for the user to confirm the position by her eyes, the pad can be fitted at the right position by detecting a precise fitting point.

Further in the pad according to the present invention, the side portion in the longitudinal direction of the support sheet includes the peripheral edge portion where the mini sheet piece can be bonded as well as the peripheral edge portion of the pad.

(7) The interlabial pad according to (6) comprising a body-side-folded portion folded upward towards the body side along the substantial center line in the longitudinal direction of the interlabial pad and hem portions continuously sloping from both side ends of the body-side-folded portion, wherein the mini sheet piece is bonded on the garment side of the hem portion.

In the interlabial pad according to the present invention, as illustrated in Fig. 12, preferably the absorbing sheet portion (the cover sheet 41 a, 41 b and the absorbent body 43a, 43b) comprises a body-side-folded portion 44c and a hem portion 44d. The body-side-folded portion 44c is structured in a mountain-like shape toward the body side direction along the substantial centerline 46 in the longitudinal direction of the interlabial pad 44. The hem portion 44d continues from both side ends of the body-side-folded portion 44c. The mini sheet piece 52 is bonded on the garment side of the hem portion 44d.

By the structure described aforesaid, the ostium vaginae contact area 44a can easily be inserted into the deep portion between the labia and a whole shape of the interlabial pad 44 is formed in a shape easily capable of exerting a holding force of labia. Therefore the pad can provide the user with good contact state and a wear feeling. Further, the finger is inserted into the finger insertion opening 53 with the finger cushion along with the central folding line of the folded portion 44c so that the interlabial pad 44 is held. Thereby, the interfabial pad 44 can be guided correctly toward the labia which is difficult to be confirmed by her eyes and be easily put thereon.

In this embodiment, it is sufficient for the interlabial pad to have the absorb sheet portion (i.e., the cover sheet 41a, 41b and the absorbent body 43a, 43b) bent to form a mountain-like shape, as illustrated in Fig. 12. However, the support sheet 42 may be folded together with the absorb sheet portion within the scope of the present invention.

(8) The interlabial pad according to any one from (1) to (7), wherein the pad is an interlabial pad which is used together with a sanitary napkin.

There have been users to use some sanitary napkins (as referred to a napkin in the following description) by overlaying one after another in the case of a large volume of menstrual blood. However, there has been a problem of a bad wear feeling such as a stiff feeling and it is easily to find such napkins (noticeable) from the outer of the garment. Since a plurality of napkins are overlaid in areas other than near the ostium vaginae which is the only place where overlaying is needed, rash or stuffy feeling may be caused. However, if the pad and the napkin are used together, they are overlapped only around the ostium vaginae. Thereby, the problem can be solved. Further, only the pad can be replaced without replacement of the napkin so that the user does not have to carry napkins of a noticeable size.

(9) The interlabial pad according to any one from (1) to (8), wherein the interlabial pad is a pad for an incontinence.

In the interlabial pad according to the present invention, the pad can be used for incontinence absorb pad. Since the ostium vaginae where the menstrual blood is discharged and an urethral meatus where urine is discharged are located between labia, the interlabial pad used between the labia according to the present invention can also absorb urine. As described hereinbefore, the pad according to the present invention can absorb urine around labia, especially around the urethral meatus so that an absorbing pad for incontinence, especially for a light incontinence, may be obtained.

(10) The interlabial pad according to any one from (1) to (8), wherein the interlabial pad is a pad for absorbing vaginal discharge.

In accordance with the present invention, the interlabial pad can be used for a pad absorbing vaginal discharge. Since the interlabial pad is used between labia and can absorb the excretion other than the menstrual blood from the ostium vaginae, it can be used for such use (for absorbing the vaginal discharge).

As described above, the pad can absorb the vaginal discharge in order to decrease a uncomfortable feeling of the user, and is useful for the user who is not menstruating.

(11) A wrapping body comprising the interlabial pad according to any one from (1) to (10), wherein the interlabial pad is contained in a wrapping container for individual wrapping.

Here, the sanitary napkin may include not only the napkin for absorbing the menstrual blood in the market but also the sheet absorbing the vaginal discharge.

If the interlabial pad is packaged individually, each pad can be carried (by every individual wrapping). As compared with a plurality of pads contained in one wrapping container, the pad can be easily carried and can be handled simply while the pad can be kept in a sanitary condition.

(12) A wrapping body comprising the interlabial pad according to any one from (6) to (10); wherein the pad is contained in a wrapping container for individual wrapping; and wherein the wrapping container has an unwrapped opening; and wherein the pad is so contained in the wrapping container that the finger insertion opening faces toward the unwrapped opening of the wrapping container.

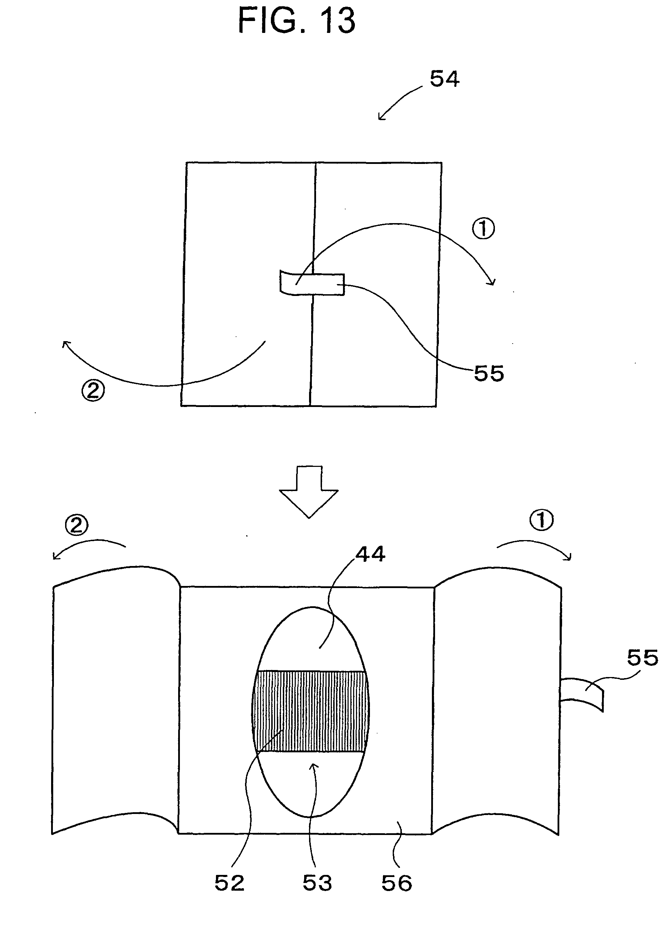

As illustrated in Fig. 13, to provide the finger insertion opening facing toward the unwrapped opening of the wrapping body, means that the pad is contained in a manner that the mini sheet piece 52 and further the finger insertion opening 53 can be exposed to the outside so that a finger can be immediately inserted into the finger insertion opening 53 when the wrapping body 54 is opened. For example, as illustrated in Fig. 13 and Fig. 14, the wrapping body 54 can be opened by pulling a tub tape 55 to the right side of the drawing and the tape 55 is provided at the upper surface side of the wrapping container 56. The finger insertion opening 53 is exposed to the opening of the wrapping body so as to open towards the unwrapped opening. Therefore, the user can insert the finger immediately into the opening 53.

(13) A wrapping body according to (12); wherein the interlabial pad is so contained in the wrapping container that the mini sheet piece is folded upwardly toward the garment side direction along the substantial center line in the longitudinal direction of the interlabial pad.

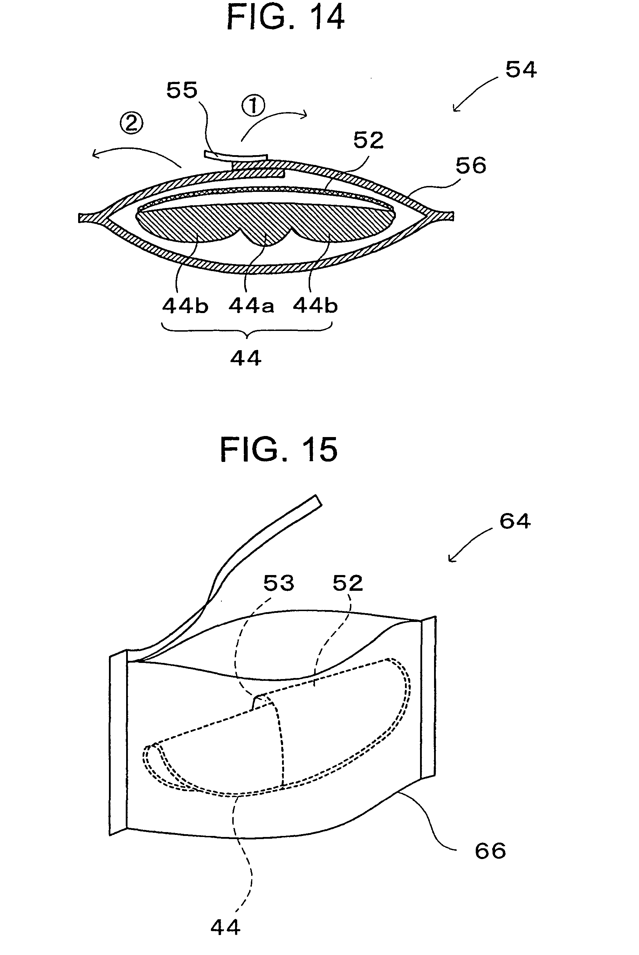

The meaning that the mini sheet piece is folded upwardly toward the garment side direction, may include that the sheet is completely folded to make a convex portion toward the garment side. As illustrated in Fig. 15, the sheet piece is inflected upwardly to make a convex portion toward the garment side. As described hereinbefore, the interlabial pad 44 is contained in the wrapping container 66. thereby, the folded finger insertion opening 53 naturally opens at the time of uwrapping the wrapping container 66. Therefore, the user can locate easily the portion where she inserts her finger so that she can wear the pad more quickly and easily.

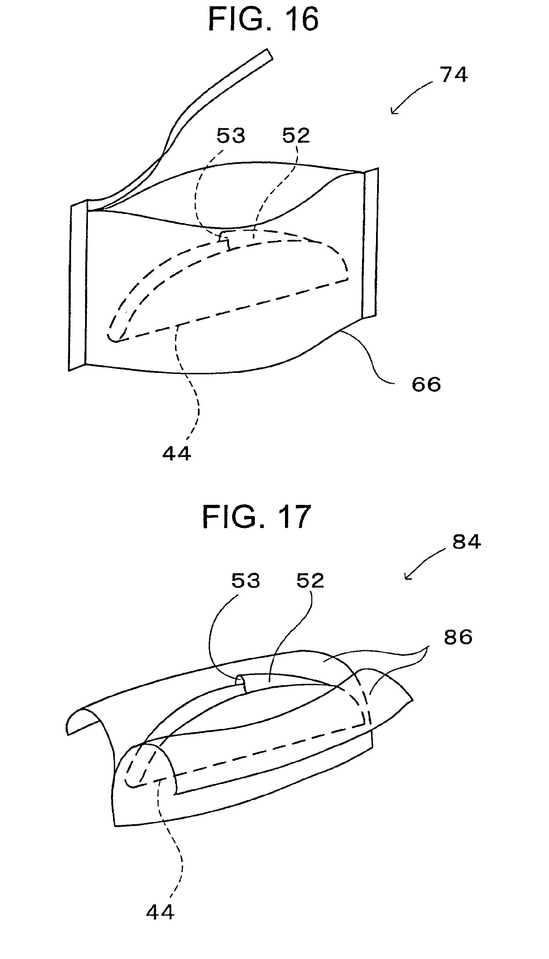

In this embodiment, the mini sheet piece may be in a mountain-like shape and it is not required to make whole pad in a mountain-like shape. Therefore, as illustrated in Fig. 15, in the wrapping body 64, the whole interlabial pad 44 is folded in a mountain-like shape and contained in the wrapping container 66. Further, as illustrated in Fig. 16, in the wrapping body 74, only the mini sheet piece 52 is folded in the mountain-like shape, while the main body of the interlabial pad 44 may be folded in a V-shape with the top opening of the V-shape toward the garment side and be contained in the wrapping container 66.

Further the method of unwrapping the wrapping container is not limited specifically. As illustrated in Fig. 15 and Fig. 16, the wrapping container may include the wrapping container 66 having an upper end cut to unwrap the pad. Further as illustrated in Fig. 17, the wrapping container may include a wrapping container 86 to be unwrapped from the top end and then to the left and right sides in a manner of a pair of folding doors.

### Brief Description of the Drawings

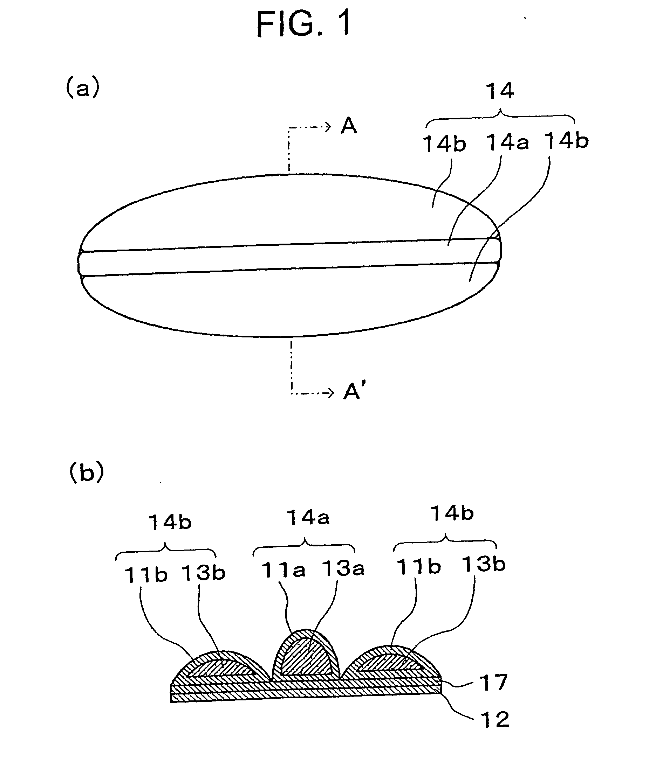

Fig. 1 is a cross sectional view of a structure of an interlabial pad according to the present invention that (b) shows a top view of the pad and (a) shows a cross sectional view along line A-A' of (b).
Fig. 2 is a perspective view of a structure of a conventional interlabial pad.
Fig. 3 is a perspective view of a structure of a conventional interlabial pad.
Fig. 4 is a perspective view of a cross section of the interlabial pad according to the present invention and (a) shows the normal condition of the pad and (b) shows a folded condition of the pad.
Fig. 5 is a top view of the structure of the interlabial pad according to the present invention.
Fig. 6 is a top view of the structure of the interlabial pad according to the present invention.
Fig. 7 is a top view of the structure of the interlabial pad according to the present invention.
Fig. 8 is a top view of the structure of the interlabial pad according to the present invention.
Fig. 9 is a perspective view of the structure of the interlabial pad according to the present invention.
Fig. 10 is a perspective view of the structure of the interlabial pad according to the present invention.
Fig. 11 is a perspective view of the structure of the interlabial pad according to the present invention.
Fig. 12 is a perspective view of the structure of the interlabial pad according to the present invention.
Fig. 13 is a process diagram of a way of opening a wrapping body according to the present invention.
Fig. 14 is a cross section view of a structure of the wrapping body according to the present invention.
Fig. 15 is a perspective view of a structure of the wrapping body according to the present invention.
Fig. 16 is a perspective view of a structure of the wrapping body according to the present invention.
Fig. 17 is a perspective view of a structure of the wrapping body according to the present invention.

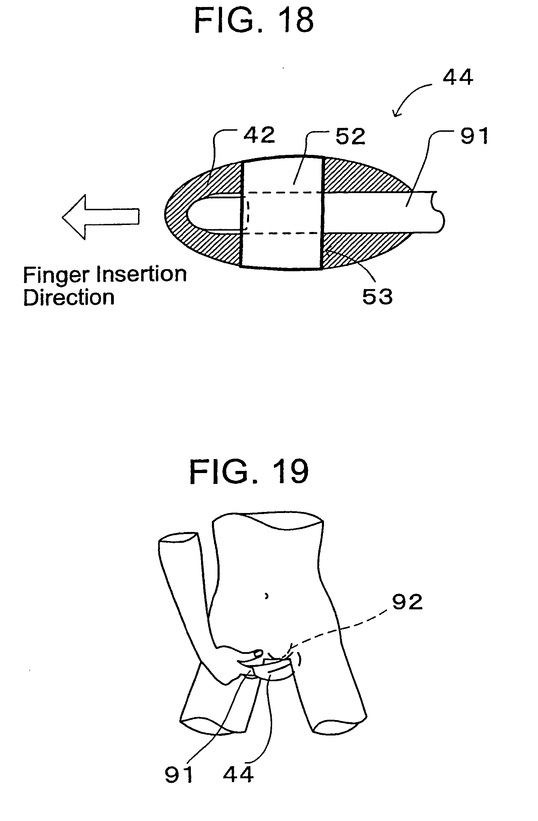

Fig. 18 is a top view of an embodiment of a mini sheet piece of the interlabial pad according to the present invention.
Fig. 19 is an explanation view of a way of wearing the interlabial pad according to the present invention.

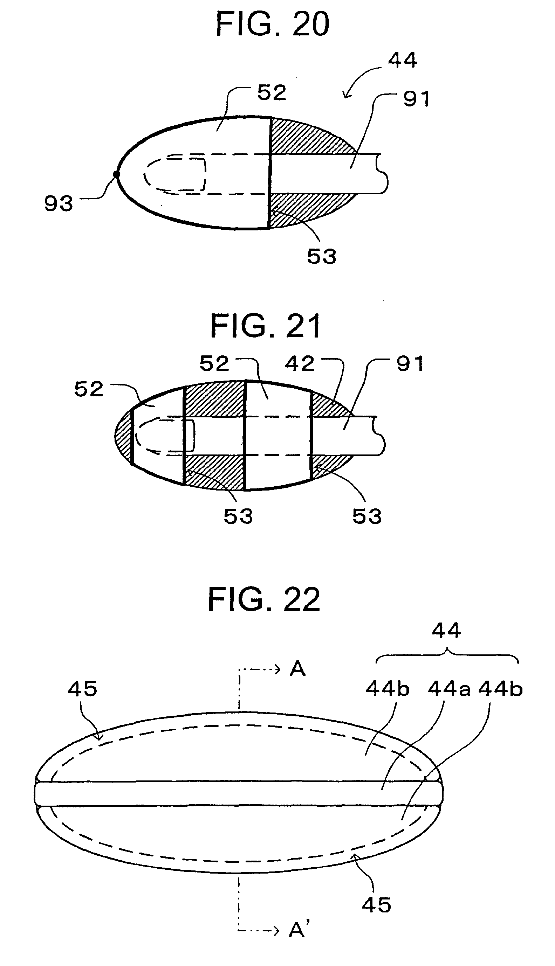

Fig. 20 is a top view of an embodiment of a mini sheet piece of the interlabial pad according to the present invention.
Fig. 21 is a top view of an embodiment of a mini sheet piece of the interlabial pad according to the present invention.
Fig. 22 is a top view of the structure of the interlabial pad according to the present invention.

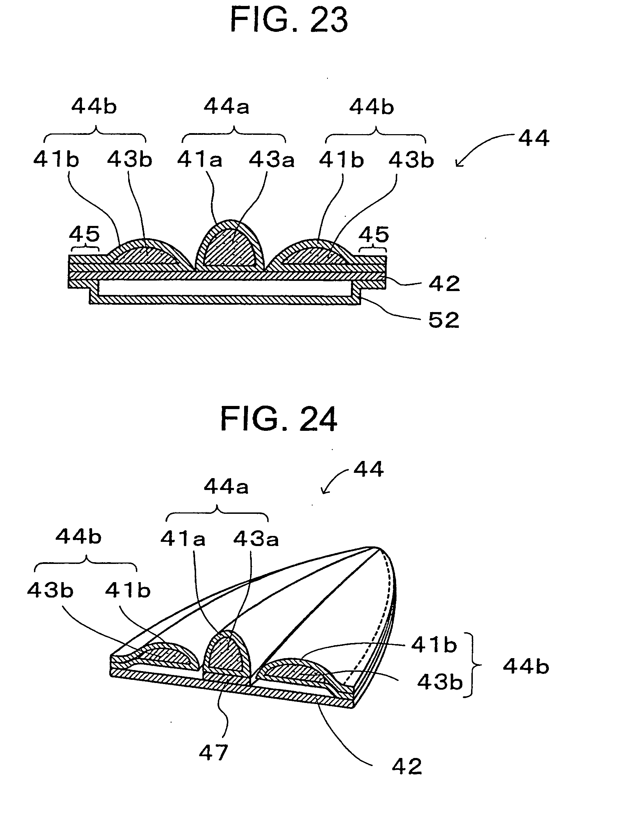

Fig. 23 is a cross section view along line A-A' of the interlabial pad of Fig. 22.
Fig. 24 is a perspective view of a section of the interlabial pad according the present invention.

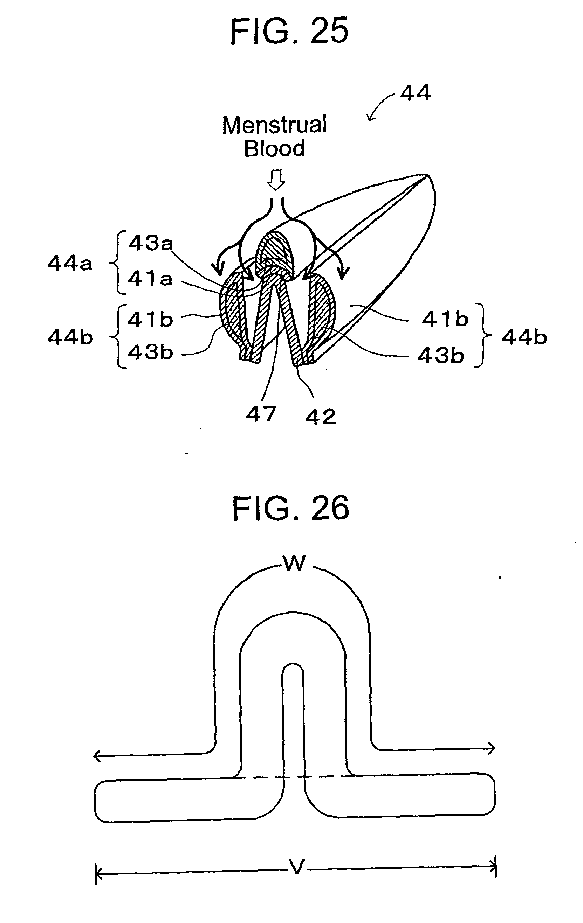

Fig. 25 is a perspective view of a folded state of the interlabial pad of Fig. 24.
Fig. 26 is an explanation view of a size in a lateral direction of the interlabial pad.

### Best Mode of Carrying Out the invention

The preferable embodiment of the interlabial pad in accordance with the present invention will be described with reference to accompanied drawings. In the specification of the present invention, the word "bulkiness" means a size in the direction which the area of the pad projects to contact an ostium vaginae, "the width" means a size in the lateral direction of the pad and "the length" means a size in the longitudinal direction of the pad.

Fig. 1 is a cross section view of a structure of an interlabial pad 14 according to the present invention that (a) shows a top view of the pad and (b) shows a cross sectional view along line A-A' of (a).

### [(A) Structure of a basic interlabial pad]

As illustrated in Fig. 1(a) and (b), in the basic interlabial pad 14, an adhesive 17 divides an absorb sheet portion of the upper part of the pad from a support sheet 12 backing the absorb sheet portion. Further the absorb sheet portion comprises strip-shaped absorbing areas 14a and 14b which respectively contact an ostium vaginae and an interlabial inner wall. The areas 14a and 14b are comprised of cover sheets 11a, 11b respectively by which absorbent bodies 13a, 13b are wrapped to absorb the menstrual blood.

The whole shape of the interlabial pad 14 is not limited as far as it fits to the labia. It is preferable to form the pad in a substantial oblong shape, for example, to an elliptic type, an ovoid type, a gourd-shape, a drop-shape and the like. It is preferable to determine the size of the interlabial pad 14 in consideration of easy fitting to the labia. In view of them, preferably the length of the pad is 60 to 150 mm, more preferably 80 to 120 mm. Further it is preferable that the length in the lateral direction of the pad is a range from 10 to 60mm in appearance, more preferably from 20 to 40mm. In case that the length in the lateral direction is longer than 60mm, the area exceeding from the labia may be rubbed by the femoral region and the like of the person, and resultantly the friction which is caused by rubbing, will overcome the holding force of both labia and there is a fear of dropping off the interlabial pad. In case of the length in the lateral direction of the pad being shorter than 10mm, the area of the pad capable of interposing between labia is decreased. Therefore the contact area with labia surface is also decreased and there is more fear of dropping off the interlabial pad from labia. The word " in appearance " means that the length is the minimum distance between two points (corresponding to V in Fig. 26). Since in the production process, there is a case that the distance tracing the uneven form, that is, the distance between two points in a flat condition of the pad which the uneven shape is provided is treated as a substantial distance (corresponding to W in Fig. 26), the distance is defined very carefully. In case of wrapping the absorbent body 13a, 13b in the cover sheet 11a, 11b, to prevent the bad wear feeling caused by the hardened peripheral edge of the absorbent body, preferably the size of the absorbent body 13a, 13b wrapped by the cover sheet 11a, 11b and/or by a support sheet 12 is structured to be 2 to 10mm smaller than the outline of the cover sheet 11a, 11b.

In the interlabial pad 14, the ostium vaginae contacting area 14a is formed to be one of the strip-shaped absorbing area at the center of the absorbing sheet portion and the labia inner wall contacting area 14b is also disposed to be one of the strip-shaped absorbing area at both side portions of the absorbing sheet portion. For an object of absorbing most menstrual blood discharged from the ostium vaginae firstly, as shown in Fig. 1 (b), it is preferably to form the ostium vaginae contacting area 14a in bulkiness more than the labia inner wall contacting area 14b.

In Fig. 1, the interlabial pad 14 comprises the area 14a to contact the ostium vaginae, and the area 14b to contact the labia inner wall, in which the absorbent body 13a, 13b is wrapped by the cover sheet 11a, 11b, and these areas 14a, 14b are bonded to the support sheet 12 by the adhesive 17.

For the adhesive 17 used for the pad, a pressure sensitive adhesive made up mainly of a synthetic rubber such as non water soluble styrene -ethylene-butadiene-styrene block copolymer (SEBS), styrene-butadiene-styrene block copolymer (SBS), styrene-isoprene-styrene block copolymer (SIS) and the like, the heat sensitive adhesive made up mainly of a heat flexible resin such as ethylene-vinyl acetate copolymer (EVA) and the like, the adhesive made up mainly of water soluble heat flexible resin (such as poly vinyl alcohol (PVA)) , water sensitive gel made up mainly of a starch glue, or acrylic acid and comprised of the chemical simulation, the felxibilizer or a water included therein, non water sensitive gel made up mainly of a silicone and comprised of the chemical stimulation and the flexibilizer included therein, are eligible. It may select to dispose the adhesive from a surface type, a dot type, a mesh type, a stripe type and the like.

In the interlabial pad 44 illustrated in Figs. 24, 25, the ostium vaginae contacting area 44a is adhered on the support sheet 42 by the adhesive 47 and it is designed that via the cover sheet 41a, 41b, the labia inner wall contact area 44b is connected only the periphery of the interlabial pad 44 with the support sheet 42. In the interlabial pad, labia inner wall contact area 44b and the support sheet 42 are not full adhered with each other by the adhesive, as shown in Fig. 25, at the time of wearing it, a pocket-type clearance is formed between the area 44b and the sheet 42. Therefore the menstrual blood which cannot be absorbed in ostium vaginae contact area, can rapidly flow into the clearance so as to be absorbed from both sides of the labia inner wall contact area 44b and the clearance side. That is, in comparison with the interlabial pad 14 illustrated in Fig. 1, the rate of absorbing effect can be increased.

### [Cover sheet]

For the cover sheet, the sheet-typed material having a liquid permeable structure of a woven or non woven fabric are eligible and is not limited to the specific one. For the woven or non woven fabric material, both natural fabric and chemical fabric can be used. For example of the natural fabric, cellulose such as a grinding pulp and cotton is eligible. For example of the chemical fabric, a regenerated cellulose such as a viscose rayon and a fibril rayon, semi synthetic cellulose such as acetate and triacetate, a heat flexible hydrophobic chemical fiber which is treated in hydrophilic are eligible. For a heat flexible hydrophobic chemical fiber, a single fiber such as polyethylene (PE), polypropylene (PP), polyethylene terephthalate (PET), a fiber comprised of a graft polymerization of PE and PP, or a compound fiber of which core material is PP or PET and a myelin part is PE are eligible.

Furthermore, especially in case of using the non woven fabric, a web forming may be manufactured by a single dry method (a card method, a spun bond method, a melt blown method, a air laid method, a through air method, a point bond method and the like) or a single wet method and the like, or by a combination of plurality methods thereof. For a method of bonding, a thermal bonding, a needle punch, a chemical bonding and the like are eligible, however it is not limited to these methods. Further a spun lace that is formed in a sheet type by a water flow confounding may be eligible.

Among the materials, considering the liquid mobility from the inner face of the labia, chemical stimulation by an activator, and adhesion with the inner wall of the labia, it is preferable to laminate rayon with 1.1 to 4.4 dtex fineness and 7 to 51 mm fiber length by 40 to 80 % of a total specific weight per unit area on the body face side, and to laminate a mixture of rayon with 1.1 to 4.4 dtex fineness and 7 to 51 mm fiber length by 14 to 42 % of a total specific weight per unit area and PET with 1.1 to 4.4 dtex fineness and 7 to 51 mm fiber length by 6 to 18 % of a total specific weight per unit area on the garment face side. After laminating them so that the total specific weight per unit area of the two layers becomes 20 to 60 g/m², the fibers are entangled by water - flow interlacing treatment and then dried to prepare spun lace non woven fabric with the thickness of 0.13 to 0.50 mm. The spun lace non woven prepared as described is preferable. At this time, by mixing PET on the garment face side, bulkiness can be easily maintained even if the water permeable sheet becomes wet. Therefore, adhesion to the inner wall of the labia can be maintained.

Since for transferring the menstrual blood into the absorbent body, the capillarity and the water permeability is required on a part of contacting area with ostium vaginae of the cover sheet, it prefers to use a porous non woven fabric sheet having a rate of hole area from 5 to 60% and a hole area of 0.28 to 4.9mm².

While in addition to the capillarity to transfer the menstrual blood into the absorbent body, the improved contact ability with the labia inner wall is required to the part comprising the area contacting with the labia inner wall, and it prefers to use the non woven fabric sheet, which has the surface without the unevenness and is not provided with the porous.

### [Absorbent body]

The absorbent body may be capable of keeping and absorbing a liquid (the menstrual blood) and preferably may be bulkiness, may not be difficult to lose the shape and may be less chemical stimulation. For the material of the absorbent body, cellulose (natural pulp, chemical pulp or natural cotton and the like), a regenerated cellulose (a viscose rayon and a fibril rayon and the like), a semi synthetic cellulose (acetate and triacetate and the like), a particle water-absorbent polymer, a fiber-type water-absorbent polymer, a chemical fiber (a heat flexible hydrophobic chemical fiber which is treated in hydrophilic and the like) and a hydrophobic resin can be used in single or can be mixed.

The method of forming these materials to the absorbent body is not limited to the specific one, however, for example, the materials is manufactured into the sheet type by an air laid method, a melt blown method, a spun lace method and a paper making and the like. Concretely, the air laid pulp (or water-absorbent polymer is mixed therewith), the melt blown non woven fabric treated into hydrophobic, the spun lace non woven fabric mainly comprised of hydrophobic fiber, tissue, cellulose foam, a continuous foam of the synthetic resin and the like can be used. Furthermore it is possible to grind these sheet-typed materials and thereafter to form them into the absorbent body again.

It is preferable for the absorbent body, although any material can be used as long as it is capable of absorbing and holding liquid (fluid), to be bulky, hard - to - be deformed, less chemically stimulant, and highly flexible to fit into the labia. Specifically, a non woven sheet in which, 50 to 150 g/m² of pulp selected from the range of the fiber length of 1 to 10 mm is laminated on the garment face side and, on the body face side, 150 to 250 g/m² of a mixture obtained by mixing 60 to 90 % of rayon with 1.1 to 4.4 dtex fineness and 20 to 51 mm fiber length with 40 to 10 % of natural cotton by this mixing ratio is laminated, which then to be formed into a sheet by dotted embossing to have 2 to 10 mm bulkiness, and more preferable to have 3 to 5 mm bulkiness. Thereby, liquid can be easily transferred from the body face side to the garment face side resulting in the improvement of the absorbing and holding capacity. Furthermore, by providing a mesh spun lace non woven fabric of rayon with 1.1 to 4.4 dtex fineness and 25 to 51 mm fiber length by a specific weight per unit area of 15 to 40 g/m², the liquid transferred from the body face side can be diffused by the mesh spun lace to be induced to almost all over the region of the pulp layer. Therefore, more liquid can be effectively absorbed.

### [Support Sheet]

In case of using the water permeability material, the same material, which is used for the cover sheet, is eligible. In this case, it prefers to use the pad together with a sanitary napkin (a pad used together with the sanitary napkin).

Further if non-water permeability material is used, the menstrual blood, which is kept in the absorbent body, is prevented from a leak out of the interlabial pad. Furthermore, the pad can be made of water vapor permeability material so that the sweat and the discomfort can be decreased in wearing the pad. To apply materials of non-water permeability, non-water permeability film such as a thin filmed synthetic resin of PE, PP and the like, a porous film comprised that a synthetic resin is filled with inorganic filler and provided with an extension treatment, a laminate film compound of a paper or non woven fabric and non-water permeability film, water repellent, a porous resin film is connected on a rear surface of the non woven fabric of a spun bond or a spun lace which are treated with a water repellent, are eligible. Further for a method of providing ventilation on non-water permeability sheet, it is eligible to form a capillary having 10 to 30% rate of the hole area and a pore size of 0.1 to 0.6 mm toward the absorbent body.

More concrete example of applying non-water permeability materials, a film mainly of a low density polyethylene (LDPE) is eligible which is obtained from a range of a density 0.900 to 0.925g/cm³, in amount from 15 to 30g/m² by mass, based on the total mass per unit area of the composition. The flexibility not to hurt a wear feeling is considered. More preferably, to decrease a fear of dropping the interlabial pad from the labia due to the high friction, if non-water permeability sheets are contacted with each other, or with the pad which is used together, or with the under wear in wearing the pad between the labia, the film is treated through an embossing process to provide the convex upheaval portions, so that a contact ratio is decreased to reduce friction drag.

### [Mini Sheet Piece]

It is preferable that a material used for the mini sheet piece is selected in consideration of having an enough strength against a damage by the inserted finger. It is possible to select one or more, which are laminated, from a sheet-typed non woven fabric, an elastic and expand non woven fabric, a film, a foam film, an elastic and expand film, a foam sheet, a tissue paper and the like.

Preferably the breaking strength in the lateral direction of the mini sheet piece, which is structured by materials described hereinbefore, is 0.1N/10mm, more preferably from 0.1 to 1.0 N/10mm. This breaking strength is a value per a width of 10mm, which is evaluated by a tensilon testing that the mini sheet piece is held with a chuck distance of 100mm and pulled by a velocity of 100mm/min.

In consideration of the above-described condition, concretely the film which has a thickness from 15 to 30µm and is mainly composed of LDPE resin having a density of 0.920g/cm³, is eligible. While for easily putting the finger in and out of the opening through of the mini sheet piece, it is preferably to select the foam film which is mainly composed of LDPE resin having a density from 0.915g/cm³ and on which a capillary which has a bulkiness from 0.3 to 1.0mm and a pore size from 0.3 to 1.5mm, is formed at the rate of hole area from 15 to 60%.

It is possible to use the material having an extensibility or an elastically expand to the lateral direction of the support sheet for inserting the finger into the mini sheet piece regardless of the finger size of the user.

For providing the extensibility to the mini sheet piece, an extensible spun bond non woven fabric may be used. The fabric has a stress from 0.1 to 0.5N/25mm in 5 % extension in case of a constant speed extension by a speed of testing of 100mm/min with a knob distance of 100mm. Further to provide the mini sheet piece with an elastic expansive property, a fiber-shaped sheet and a film sheet using a heat flexible elastomer resin, elastic expansive materials such as these heat flexible resin and a natural rubber, independently or in combined with each other, or their combinations with inelastic expansive materials. Further it is a preferable embodiment to provide elasticity for a film mainly formed from LDPE resin by a corrugate treatment.

The length of the mini sheet piece is determined so that the user can hold the interlabial pad surely and that the user can easily put the finger into the opening. Concretely the length of the mini sheet piece is preferably at least 10 % over than the length of the interlabial pad, more preferably within a range from 10 to 80%, and most preferably within a range from 30 to 60%. The width of the opening through for inserting the finger prefers at least 20mm or more, more preferably within a range from 20 to 50mm, and most preferably within a range from 30 to 40mm.

With respect to the form of the mini sheet piece, for example, as shown in Fig. 18, it is eligible to form that along the garment face side of the support sheet 42 comprising the interlabial pad 44, the strip-typed mini sheet piece 52 is horizontally disposed in the' lateral direction of the interlabial pad 44. In this example, the mini sheet piece 52 is fixed at both side ends of the interlabial pad 44 and the opening through 53, that is the opening for inserting the finger, is formed toward the longitudinal direction of the interlabial pad 44.

In the embodiment, when the finger 91 is inserted into the opening through 53 with contacting a ball of a finger 91 to the support sheet 42, as illustrated in Fig. 19, the longitudinal direction of the interlabial pad 44 and a direction of pudendal slit 92, face in the same direction. Therefore the interlabial pad 44 can be pushed into the inside of the labia by a ball of the finger 91 so that it is attached certainly. Further the mini sheet piece may be formed as illustrated in Fig. 20, that the support sheet 42 which comprises the interlabial pad 44, is completely covered from the near central portion in the longitudinal direction to an edge end 93 in the longitudinal direction of the interlabial pad 44. The embodiment described hereinbefore is preferable for a sanitary handling of the pad so that the finger end 91 is prevented from exposing out of the mini sheet piece 52 and non-contact condition of the menstrual blood and the finger 91 can be maintained.

For example, as illustrated in Fig. 21, the end of the finger 91 is also prevented from exposing out of the mini sheet piece 52 by the interlabial pad 44 which a plurality of strip-shaped mini sheet pieces 52 are formed at distance, thereby the effect of the sanitary handling can be achieved as same as the interlabial pad 44 as shown in Fig. 20.

Fig. 22 is a top view of the interlabial pad and Fig. 23 is a section view along line A-A' of the interlabial pad of Fig. 22. For example, in case of providing the mini sheet piece, as illustrated in Fig. 23, it prefers to form the clearance to insert the finger preliminarily by somewhat rising the mini sheet piece 52 from garment face side of the support sheet 42.

In this embodiment, the width of the mini sheet piece 52 is formed somewhat larger than that of the interlabial pad 44 and the piece 52 is bonded with the periphery edge portion 45 of the interlabial pad 44 in somewhat loose condition not to contact with the garment face side of the support sheet 42. The method of fixing the pad is not limited specifically, for example, the method of pressure sensitive hot melt adhesive, heat sensitive hot melt adhesive, heat seal, ultra sonic seal and the like will be eligible. The application of the adhesive can be selected from, for example, a surface-shaped, a line-shaped, a spiral-shaped, a dot-shaped and the like.

In the interlabial pad 44 illustrated in Fig. 23, the side end of the mini sheet piece 52 is bonded with the periphery edge portion 45. In this embodiment, to prevent the periphery edge portion 45 from the stiff wear feeling of the interlabial pad 44, preferably the width of connecting portion is set in a range from 2 to 5 mm. Further the mini sheet piece 52 is connected inwardly (toward the side of the central portion) of the interlabial pad 44 from the periphery edge portion 45, thereby preferably a soft feel of materials is provided on the periphery edge so as to increase a good wear feeling. In this embodiment, it is preferred that the mini sheet piece 52 is adhered on the support sheet 42 by hot melt-typed adhesive.

For bonding between the mini sheet piece and the support sheet with an adequate strength, in respect of the bonding strength, it is preferably to set the breaking strength in a lateral direction of the pad in a range from 0.3 to 1.2 N/10mm. This breaking strength is a strength per a width of 10mm and a value which is evaluated by a tensilon testing that the mini sheet piece held on the upper chuck and the support sheet held on the lower chuck with a chuck distance of 20mm,are pulled by a velocity of 100mm/min.

To achieve an easy identification of the mini sheet piece by the user, the mini sheet piece can be adjusted to have a different color or design, chromaticity from the support sheet by coloring or printing patterns.

### [Wrapping Container]

The conventional wrapping container can be used for wrapping the interlabial pad according to the present invention. For example, non woven fabric comprised of PE, PP, PET and the like, a film having a thickness from 15 to 60µm, a paper or laminate materials treated by a lamination of these materials is eligible.

In consideration of a soft feeling, the inner side face of the wrapping container is preferably comprised of a crepe tissue in a range from 15 to 50g/m² by total mass of composition, wet spun lace non woven fabric in amount from 15 to 70g/m² by total mass made of a composition of a cotton and a pulp, which includes a cotton at least 10% by mass, a spun lace non woven fabric in a range from 20 to 70g/m² based on the total mass of a composition which includes a rayon at least 30% by mass, or melt blown non woven fabric comprised of PP in amount from 20 to 50g/m² by total mass. Further it is also preferable to comprise the container by a composed non woven fabric which is comprised a melt blown non woven fabric in a range from 5 to 20g/m² by mass is held between the spun bond non woven fabric in amount from 6 to 10g/m² by total mass of composition. While it is preferable to comprise the outer surface side of the wrapping container in consideration of water durable pressure by a film comprising PE in amount from 10 to 30g/m² by total mass of composition or a porous plastic sheet having a rate of hole area from 10 to 30% and in amount from 15 to 30g/m² by total mass of composition.

The inner and outer face side materials of the wrapping container are laminated in unit by a conventional method of a hot melt adhesive, heat embossing or an ultra sonic sealing and the like. In this embodiment, it is preferable to cover the hot melt adhesive in a spiral-shaped or a line-shaped with application coverage from 3 to 10g/m² and with a rate of applicant area from 5 to 40%. In case of a heat embossing or an ultra sonic sealing, the adhesive is applied in a line-shape, a dot-shape or a cross line-shape and the like in a sealing area from 5 to 20% in consideration of the feeling of the laminate material.

### [(B) Structure of the interlabial pad provided with biodegradable rate, water dispersible and water soluble]

Preferably the interlabial pad is comprised of a material of biodegradable rate and/or a material of water dispersible and/or a material of water-soluble. After using the pad comprised of these materials, it can be disposed into a toilet to flush, thereby the destruction of the pad can be easily and sanitarily achieved and the garbage in a toilet can be decreased.

In this Specification, "biodegradable" means that a substance is decomposed into gas such as carbon dioxide or methane, water, and biomass under anaerobic or aerobic condition according to the natural process under the existence of bacteria represented by actinomycetes and other microbes, and also means that the biodegradability (biodegradable rate and biodegradable degree) of the substance equals to a material naturally generated such as fallen leaves or a synthetic polymer generally recognized having the same biodegradability under the same environment. "Water dispersible" has the same meaning as water soluble. It means a characteristic in which, while having no influence when used in a limited amount of moisture (menstrual blood), in a large amount of water or water current, the fabric is easily dispersed into small pieces at least to a degree where an ordinal toilet plumbing is not clogged. "Water soluble" is a characteristic in which, while having no influence when used in a limited amount of moisture (menstrual blood), the fabric is soluble in a large amount of water or water current.

### [Cover Sheet]

Any of natural fiber and chemical fiber can be used for materials of the cover sheet to achieve biodegradable rate, water dispersible and water-soluble. For example of natural fiber, there are cellulose such as a grinding pulp and cotton and air laid pulp and the like which is chemically composed by water-soluble resin. For examples of chemical fiber, there are regenerated cellulose such as rayon, fibril rayon, PE, PP, PET, materials which is hydrophilic treated to the chemical fiber such as ethylene-vinyl acetate copolymer, so-called biodegradable rate fiber such as poly lactic acid, polybutylenesuccinate and the like. Further water-soluble carboxymethylcellulose and polyvinyl alcohol are eligible for use. In these materials, it prefers to use cellulose such as a pulp and cotton, regenerated cellulose such as rayon and the like and so-called biodegradable rate fiber such as poly lactic acid.

Materials described hereinbefore can be used independently or be mixed by forming a web or a non woven fabric. For web forming biodegradable rate fabric such as poly lactic acid or polybutylene succinate and the like, any of a card method, a spun bond method, a melt blown method or a dry method or a wet method by an air laid method, or a combination of these is eligible. For a method of bonding, a thermal bonding, a needle punch bonding, a chemical bonding are eligible, however it is not limited to these method.

Further a spun lace formed in a sheet-shaped by a water flow confounding is eligible.

By way of example, a forming method for making a water dispersible fabric may comprise making a water degradable paper in a sheet form utilizing hydrogenbond among fibers. A method for making a water dispersible paper may comprise binding fibers with a water soluble binder and forming the fibers into a sheet. A method for making a water degradable paper in a sheet form may comprise confounding the fibers.

It is preferable to have a length range of the fiber from 2 to 51mm and it is more preferable further from 2 to 10mm to achieve good water dispersibility. It is preferable to select the fiber finess (thickness) range from 1.1 to 4.4 dtex in order to have both the water dispersibility and strength enough not to be broken during the use. In particular, it is preferable to select the finess (thickness) from 1.1 to 3.3 dtex if rayon is used. If the finess is less than the ranges described above, the water dispersibility may be achieved. However, in a dry condition, the fiber may easily become fuzzy and lose such fuzz. If fineness is more than the value described above, water dispersible is extremely deteriorated.

Preferably the amount of the cover sheet is selected from 20 to 60g/m² by mass based on total mass of composition. Further it is required that the breaking strength of the cover sheet is 800mN/25mm at least in both vertical and horizontal directions and preferably is from 1000 to 7000mN/25mm in consideration of the softness in wearing the pad (the breaking strength of the sheet evaluated from the constant speed extension by a speed testing 100mm/min in a condition of a knob distance 100mm).

For further concrete structure of the cover sheet, a wet spun lace non woven fabric is eligible, that a rayon having a fineness of 1.1 to 4.4 dtex and a length from 5 to10mm and a wood pulp are mixed in a ratio from 90: 10 to 70: 30 by mass to adjust the amount from 25 to 40g/m², and the thickness from 0.2 to 0,5mm. In order to substantially improve the water permeability of the menstrual blood or to apply an good image of the menstrual blood permeability, a plurality of holes can be also provided on the sheet in configuration having a hole diameter from 0.5 to 1.5mm and a rate of hole area (a ratio of opening hole in respect of whole area) from 3 to 20%.

### [Absorbent body]

Same materials having water permeability for the cover sheet can be used for materials of the absorbent body to apply biodegradable rate ability, water dispersible and water-soluble. Further it is possible to independently use the absorbent body such as alginic acid soda, starch, carboxymethylcelluloce and the like, particle-typed or fiber-typed super absorbent polymer, or to use a form by mixing these materials with same materials for the cover sheet.

In respect of the structure of the absorbent body, the wood pulp and the like are eligible, that is laminated to the amount from 150 to 500g/m² by mass to enclose into tissue and is adjusted the thickness from 2 to 10mm by a press device. It is possible to improve the absorption capacity or keeping ability of the menstrual blood by mixing absorbent body such as starch and the like in a ratio from 5 to 30g/m².

### [Support Sheet]

For materials of the support sheet having biodegradable rate ability, water dispersible and water soluble ability, and further non-water permeability, cellulose guide materials such as methyl cellulose, hydroxyethylcellulose, carboxymethylcellulose and the like, water-soluble polymer such as polyvinyl alcohol, alginic acid, polyacrylic acid soda, polyacrylic ether, polyvinylpyrrolidone, a copolymer of isobutylene and maleic anhydride, or biodegradable polymer such as poly lactic acid, polybutylenesuccinate, starch and dextrin are eligible.

These materials can be formed in the film-typed or the sheet-typed in individual or by a mixture. At least on one surface, preferably on both surfaces of the film sheet, waterproof materials such as silicone resin can be applied or mixed. The laminate paper comprised of the non woven fabric which is treated by laminating tissue, is eligible. Further if necessary, the support sheet can be applied the coloring by mixing the mineral pigment in amount from 0.1 to 5 % by mass.

In respect of the embodied structure of non-water permeability support sheet, for example, a laminate paper is formed by a laminating treatment that a film and tissue are bonded. The film comprises poly lactic acid and tissue has a thickness from 10 to 20µm, in the amount from 15 to 20g/m² by mass based on the total mass of composition, are laminated in area ratio from 5 to 40%. The laminate paper described hereinbefore can keep non-water permeability during the pad is wet and it is preferable in preventing the digestion tank from an exceed damage.

### [Mini Sheet Piece]

In respect of materials for the mini sheet piece to apply biodegradable rate, water dispersible and water soluble, poly lactic acid, polybutylene succinate, a film comprised from PVA and the like, or materials laminated the film of these materials with tissue are eligible.

### [Wrapping Container]

For applying biodegradable rate ability, water dispersible and water soluble ability, the container may be comprised of a fiber sheet using water soluble fiber, a film using biodegradable rate resin or water soluble resin, or a laminated materials of the fiber sheet with the film, a laminated materials of the film and tissue.

### [Bonding Method]

Further for a bonding method of applying biodegradable rate ability, water dispersible, water soluble, a bonding method such as adhesion by polyvinyl alcohol and the like having water soluble or water swelling, a heat sealing, or a bonding by a hydrogen bonding, and the like can be used individually or can be used in a combination of them adequately.

### Industrial Applicability

As described hereinbefore, according to the present invention, the interlabial pad is formed as an aggregate juxtaposing a plurality of strip-typed absorbing areas. The strip-typed absorbing area includes the absorbent body, which is independently contained in the cover sheet. The area, which contacts the neighborhood of ostium vaginae of the user, and the area, which contacts the neighborhood of the inner wall of the user's labia, are formed in the strip-typed absorbing area. Therefore the menstrual blood that flows along the labia inner wall can be absorbed into the absorbent body rapidly and the shape fitness (fitting ability) in labia can be increased.

## Claims

1. An interlabial pad which is used by fitting between labia comprising:
an absorbing sheet portion facing a body side when the pad is worn, the absorbing sheet portion including an absorbent body that absorbs body fluid, and
a support sheet backing the absorbing sheet portion;
wherein the absorbing sheet portion is formed as an aggregate juxtaposing a plurality of strip-type absorbing areas in a belt shape which include independent absorbent bodies; and
wherein the strip-type absorbing areas disposed on a central portion of the absorbing sheet portion form a body fluid discharge port contact area that contacts a vicinity of a body fluid discharge port of the user; and
wherein the strip-type absorbing areas disposed on both outwardly-folded sides of the absorbing sheet portion form a labia inner wall contact area that contacts an inner wall of the user's labia.

2. The interlabial pad according to claim 1;
wherein a side end of the labia inner wall contact area comprises an arc-shaped portion; and
wherein both edge ends of the arch-shaped portion converge toward edge ends in a longitudinal direction of the body fluid discharge port contact area.

3. The interlabial pad according to claim 1 or 2;
wherein the body fluid discharge port contact area and/or the labia inner wall contact area is divided into a plurality of portions.

4. The interlabial pad according to any one of claims 1 to 3, comprising:
a groove portion disposed between the body fluid discharge port contact area and the labia inner wall contact area, or between the body fluid discharge port contact area divided into a plurality of portions and the labia inner wall contact area divided into a plurality of portions.

5. The interlabial pad according to any one of claims 1 to 4, comprising:
an inner sheet having water permeability or water-absorbability and being inserted between a garment side face of the absorbing sheet and a body side face of the support sheet.

6. The interlabial pad according to any one of claims 1 to 5, comprising:
a mini sheet piece provided and bonded on the garment side face of the support sheet that has one or more bonding portions at each side portion in a longitudinal direction of the support sheet and has one or more non-bonding portions in a lateral direction of the support sheet; and
wherein, between the mini sheet piece and the support sheet, at least one non-bonding portion forms a finger insertion opening through which a finger can be inserted so that an opening size of the finger insertion opening is directly maintained as large as a finger breadth in a surface direction of the support sheet.

7. The interlabial pad according to claim 6, comprising:
a body-side-folded portion folded upward towards the body side along the substantial center line in the longitudinal direction of the interlabial pad and
hem portions continuously sloping from both side ends of the body-side-folded portion,
wherein the mini sheet piece is bonded on the garment side of the hem portion.

8. The interlabial pad according to any one of claims 1 to 7;
wherein the pad is an interlabial pad which is used together with a sanitary napkin.

9. The interlabial pad according to any one of claims 1 to 8;
wherein the interlabial pad is a pad for an incontinence.

10. The interlabial pad according to any one of claims 1 to 8;
wherein the interlabial pad is a pad for absorbing vaginal discharge.

11. A wrapping body comprising the interlabial pad of any one of claims 1 to 10;
wherein the interlabial pad is contained in a wrapping container for individual wrapping.

12. A wrapping body comprising the interlabial pad of any one of claims 6 to 10;
wherein the pad is contained in a wrapping container for individual wrapping; and
wherein the wrapping container has an unwrapped opening; and
wherein the pad is so contained in the wrapping container that the finger insertion opening faces toward the unwrapped opening of the wrapping container.

13. A wrapping body according to claim 12;
wherein the interlabial pad is contained in the wrapping container so that the mini sheet piece is folded upwardly toward the garment side direction along the substantial center line in the longitudinal direction of the interlabial pad.
